# EUROPÄISCHE PATENTSCHRIFT

(11) **EP 2 874 678 B1**
(45) Veröffentlichungstag und Bekanntmachung des Hinweises auf die Patenterteilung: **20.06.2018**
(21) Anmeldenummer: 13762386.4
(22) Anmeldetag: 19.07.2013
(51) Int. Cl.: A61M 1/16

(54) **VERFAHREN UND VORRICHTUNG ZUR EINSTELLUNG DER MENGEN ODER DER PARTIALDRÜCKE ZWEIER GASE IN EINER FLÜSSIGKEIT**
METHOD AND DEVICE FOR ADJUSTING THE AMOUNTS OR THE PARTIAL PRESSURES OF TWO GASES IN A LIQUID
PROCÉDÉ ET DISPOSITIF DE RÉGLAGE DES QUANTITÉS OU DES PRESSIONS PARTIELLES DE DEUX GAZ DANS UN LIQUIDE

(30) Priorität: 20.07.2012 DE 102012106593; 22.10.2012 DE 102012110067
(43) Veröffentlichungstag der Anmeldung: 27.05.2015
(73) Patentinhaber: Thiele, Christine, 01129 Dresden (DE); Strauß, Andreas, 44801 Bochum (DE); Hohmann, Günter, 45770 Marl (DE); Aschenbrenner, Ulf, 40629 Düsseldorf (DE)
(72) Erfinder: GRANSOW, Marian, 03044 Cottbus (DE); THIELE, Christine, 01129 Dresden (DE); HOHMANN, Günter, 45770 Marl (DE); KRÜSEMANN, Bernard, 45657 Recklinghausen (DE)
(74) Vertreter: Kaufmann, Sigfrid
(86) Internationale Anmeldenummer: PCT/DE2013/100267
(87) Internationale Veröffentlichungsnummer: WO 2014/012536

(56) Entgegenhaltungen:
- EP-A1- 2 462 966
- WO-A2-03/092776
- US-A- 3 927 981
- US-A- 5 810 759
- US-A1- 2010 101 657

## Beschreibung

Die Erfindung betrifft eine Vorrichtung und ein Verfahren, mit denen die Menge oder der Partialdruck eines ersten Gases, insbesondere Sauerstoff, und der Gehalt eines zweiten Gases, insbesondere Kohlendioxid, in einer Flüssigkeit, insbesondere Blut, unabhängig voneinander auf vorgegebene Werte eingestellt werden können und welche eine automatische Regelung auf die vorgegebenen Werte erlauben.
Ist bei einem Patienten die natürliche, physiologische Funktion der Sauerstoffaufnahme in das Blut und/oder die Kohlendioxidabgabe aus dem Blut stark eingeschränkt, z.B. bei Erwachsenen mit schwerstem akutem Lungenversagen oder bei Neugeborenen und Kleinkindern mit angeborenem Lungenversagen, so wird als Standardtherapie der Gasaustausch mittels einer extrakorporalen Membranoxygenierung (ECMO) unterstützt oder ganz ersetzt. Die ECMO wird mit sog. extrakorporalen Lungenunterstützungssystemen durchgeführt.
Aus dem Stand der Technik sind Verfahren/Vorrichtungen zur Durchführung von EC-MOs (z.B. US 8,206,594 B2, DE 10 2010 004 600 A1 und US 2011/0129389 A1) bekannt, bei denen in einem Blutgasaustauscher (Oxygenator) entlang der einen Seite einer gasdurchlässigen (semipermeablen) Membran das Blut des behandelten Patienten und entlang der anderen Seite ein Prozessgas vorbeigeleitet wird, sodass aufgrund von eingestellten Partialdruckdifferenzen zwischen Blut und Prozessgas Sauerstoff aus dem Gas an das Blut abgegeben und Kohlendioxid vom Gas aus dem Blut aufgenommen werden kann. WO 03/092776 offenbart ein ECMO-System mit geschlossenem Gaskreislauf für ein Prozessgas und einem Absorber für Kohlendioxid. Aus US 5,810,759 ist ein System bekannt, bei dem die Flussrate des Prozessgases basierend auf einer Messung der Konzentration von Sauerstoff und Kohlendioxid in Blut geregelt wird. Bei pumpengetriebenen Verfahren wird dem Patienten Blut durch Kanülierung entnommen, das Blut mittels einer Pumpe durch den Oxygenator geleitet und das so aufbereitete Blut dem Patienten wieder zugeführt. Der Blutfluss kann dabei bis zu 8 l/min, der Sauerstofffluss (bei schwerstem Lungenversagen wird als Prozessgas reiner Sauerstoff eingesetzt) auf der Gasseite bis zu 10 l/min betragen. Das Prozessgas, das nach Verlassen des Oxygenators aus nicht verbrauchtem Sauerstoff und aus aus dem Blut entferntem Kohlendioxid besteht, wird an die Umgebung abgegeben (sogenanntes offenes System).

Größen zur Beeinflussung der Partialdrücke von Sauerstoff und Kohlendioxid im Blut sind neben den spezifischen Größen des Oxygenators (z.B. Membranoberfläche) die Strömungsgeschwindigkeit des Blutes (Blutfluss), die Strömungsgeschwindigkeit des Prozessgases (Gasfluss), die Zusammensetzung des Prozessgases sowie der Gesamtdruck des Prozessgases als Summe der Partialdrücke der Fraktionen.

Diese Stellgrößen beeinflussen jedoch bei offenen Systemen gleichzeitig den Sauerstoff- und den Kohlendioxidtransfer durch die Membran, d.h., die Partialdrücke von Sauerstoff und Kohlendioxid im Blut des Patienten können nicht unabhängig voneinander, sondern lediglich undifferenziert (miteinander gekoppelt) gesteuert werden. Insbesondere geht bei offenen Systemen eine Änderung der Gasströmungsgeschwindigkeit mit einer ausgeprägten Änderung des aufgrund des Strömungswiderstands des Systems entlang des Strömungsweges vorliegenden Druckabfalls bzw. Staudrucks einher.

Durch eine Änderung des Blutvolumenstroms (Strömungsgeschwindigkeit des Blutes) kann zwar eine Änderung des Kohlendioxidpartialdrucks bei einer nur geringfügigen Änderung des Sauerstoffpartialdrucks erreicht werden, wodurch prinzipiell eine weitgehend unabhängige Regelung möglich wäre. Eine Änderung des Blutvolumenstroms führt jedoch zu Kreislaufschwankungen beim Patienten, d.h., sie sollte, wenn überhaupt, nur in sehr engen Bereichen eingesetzt werden.

Eine Regelung über die Gaszusammensetzung des Prozessgases ist vergleichsweise aufwendig und fehleranfällig, zumal als zusätzliche Gerätekomponente ein Gasmischer benötigt wird.

Bei den bekannten ECMO-Verfahren ist zudem nachteilig, dass, da üblicherweise hohe Sauerstoffflüsse erforderlich sind, große Gasspeicher/Sauerstoffflaschen benötigt werden (problematisch insbesondere bei mobilen Anwendungen). Zudem können sich aufgrund des in großen Mengen an die Umgebung abgegebenen Sauerstoffs, besonders in kleinen abgeschlossenen Räumen (Rettungswagen, Rettungshubschrauber), brandfördernde Gasgemische bilden.

Aufgabe der Erfindung ist es, eine Vorrichtung zu finden, mit der in Blut die Menge oder der Partialdruck eines ersten Gases, z.B. Sauerstoff, das vom Blut aufgenommen werden kann, und die Menge oder der Partialdruck eines zweiten Gases, z.B. Kohlendioxid, das sich im Blut anreichern kann, gleichzeitig auf vorgegebene Werte geführt werden kann. Zum Erreichen der Menge oder des Partialdrucks des ersten Gases im Blut soll nur die Gasmenge erforderlich sein, die vom Blut aufgenommen wird.

Die Aufgabe der Erfindung wird durch die Merkmale des Anspruchs 1 gelöst; weitere vorteilhafte Ausführungen der Erfindung ergeben sich aus den Ansprüchen 2 bis 8. Ausgegangen wird von einem Verfahren, mit dem jeweils die Menge oder der Partialdruck eines ersten Gases auf einen ersten vorgegebenen Wert und eines zweiten Gases auf einen zweiten vorgegebenen Wert in einer Flüssigkeit, wie z.B. Blut oder Blutersatzstoff, geregelt werden kann. Die Flüssigkeit kann insbesondere eine Flüssigkeit sein, die eine oder mehrere gasbindende Komplexverbindungen enthält; wobei die Flüssigkeit insbesondere Komplexverbindungen enthalten kann, die das erste und/oder das zweite Gas unter reversibler Komplexierung (z.B. chemisch) binden können. Die Flüssigkeit kann sich insbesondere dadurch auszeichnen, dass sich die Mengen oder die Partialdrücke der beiden Gase mit der Zeit gegenläufig verändern; üblicherweise verringert sich die Menge oder der Partialdruck des ersten Gases mit der Zeit, wie z.B. Sauerstoff im Falle einer extrakorporalen Membranoxygenierung (ECMO) von Blut/Blutersatzstoff, und die Menge oder der Partialdruck des zweiten Gases, z.B. Kohlendioxid bei einer ECMO, erhöht sich mit der Zeit oder umgekehrt. Um die Mengen oder Partialdrücke der beiden Gase in der Flüssigkeit einzustellen, wird die Flüssigkeit an der einen Seite mindestens einer permeablen oder semipermeablen Trennschicht, z.B. einer Membran, und ein Prozessgas an der anderen Seite der Trennschicht vorbeigeführt, wobei das Prozessgas das erste Gas enthält oder mit dem ersten Gas identisch ist. Die Flüssigkeit kann wahlweise im Gegenstrom, Gleichstrom oder Kreuzstrom zum Prozessgas geführt sein.
Die Unterscheidung zwischen dem Partialdruck und der (spezifischen, d.h. auf ein Volumen bezogenen) Menge eines Gases in einer Flüssigkeit ist für alle diejenigen Kombinationen von Gasen und Flüssigkeiten von Bedeutung, bei denen das betreffende Gas chemische Bindungen mit in der Flüssigkeit enthaltenen Molekülen oder anderen Flüssigkeitsbestandteilen eingehen kann. So wird z.B. Sauerstoff in Blut teilweise gelöst und teilweise an Hämoglobin gebunden, sodass der Sauerstoffgehalt des Blutes entweder über den Partialdruck, z.B. mittels elektrochemischer Sensoren, oder über die Menge des an Hämoglobin gebundenen Sauerstoffs, z.B. mittels optischer Sensoren (charakteristische Spektrallinien), ermittelt werden kann. Zur Durchführung des Verfahrens wird das Prozessgas in einem geschlossenen Gaskreislauf geführt, d.h., es findet kein (permanentes) Abströmen des Prozessgases in die Umgebung statt; eventuell anfallende Verunreinigungen, wie z.B. Feuchtigkeit, können entzogen werden. Dem Prozessgas wird im Gaskreislauf das erste Gas dosiert zugeführt, um die in die Flüssigkeit übergetretene Menge zu ersetzen oder um den Druck des Prozessgases zu erhöhen, und es wird gleichzeitig dem Prozessgas die Menge des zweiten Gases, die über die mindestens eine Trennschicht aus der Flüssigkeit in das Prozessgas gelangt ist, mittels einer Absorbereinheit entzogen und anschließend abgeführt. Die Menge oder der Partialdruck des ersten Gases in der Flüssigkeit wird über den Druck des Prozessgases im Gaskreislauf auf einen ersten vorgegebenen Wert bzw. ersten Soll-Wert geführt. Die Menge oder der Partialdruck des zweiten Gases in der Flüssigkeit wird durch Vorgabe einer bestimmten Strömungsgeschwindigkeit (Umlaufgeschwindigkeit) des Prozessgases im Gaskreislauf auf einen zweiten vorgegebenen Wert bzw. zweiten Soll-Wert geführt. Eine Veränderung des Volumenstroms der Flüssigkeit ist nicht erforderlich.
Die Gasaustauschrate an der Trennschicht ist maßgeblich abhängig von den Partialdruckdifferenzen (oder Konzentrationsdifferenzen) der beiden Gasarten zwischen der Flüssigkeit und dem Prozessgas. Sammelt sich nach der Diffusion durch die Trennschicht eines der Gase lokal an, wird die betreffende Partialdruckdifferenz (der Partialdruckgradient) stark verringert und der Gastransfer limitiert. Um diesen unerwünschten Effekt zu vermeiden, sind auf beiden Seiten der Trennschicht ausreichend hohe Volumenströme (der Flüssigkeit/des Prozessgases) einzustellen, sodass beide Seiten der Trennschicht zuverlässig "freigewaschen" werden.
Je höher der Volumenstrom im Gaskreislauf ist, desto mehr Gas wird, in Abhängigkeit vom Partialdruckgradienten zwischen der Flüssigkeit und dem Prozessgas, von der Trennschicht abgeschwemmt. Für eine Verwendung des Verfahrens für die ECMO bedeutet dies, dass, je höher der Druck des Prozessgases im Gaskreislauf (und damit verbunden der Partialdruck von Sauerstoff) eingestellt wird, desto schneller und stärker kann das Blut oxygeniert werden. Je höher andererseits der Volumenstrom im Gaskreislauf eingestellt wird, desto mehr Kohlendioxid wird aus dem Blut entfernt (Decarboxylierung).

Die Verwendung des Verfahrens für die ECMO (venovenös, venoarteriell, arteriovenös) hat viele Vorteile. So können der Sauerstoffpartialdruck (bzw. die Menge des Sauerstoffs) und der Kohlendioxidpartialdruck im Blut, anders als bei bisherigen Verfahren, unabhängig voneinander eingestellt werden, wodurch Entgleisungen des Säure-Basen-Haushaltes des Blutes sicher vermieden werden. Eine Veränderung des Volumenstroms des Blutes, die regelmäßig zu Kreislaufproblemen bei den behandelten Patienten führt, ist hier nicht erforderlich.
Es kann insbesondere vorgesehen sein, dass der im Gaskreislauf vorliegende Druck kleiner ist als der in der Umgebung der Vorrichtung vorliegende Umgebungsdruck; wobei der Druck im Gaskreislauf z.B. kleiner als der Atmosphärendruck (z.B. unterhalb von 1 bar) gehalten sein kann. Insbesondere bei der ECMO, d.h. bei Blut als Flüssigkeit und Sauerstoff als erstem Gas, wobei die zur Durchführung des Verfahrens verwendete Vorrichtung als Oxygenator ausgebildet ist, ist der Betrieb des Oxygenators mit einem geringen Gasdruck vorteilhaft; wobei der Druck im Gaskreislauf insbesondere unterhalb des Blutdrucks, besonders bevorzugt unterhalb des Atmosphärendrucks gehalten ist. Ein weiterer Vorteil besteht darin, dass, im Vergleich zu bekannten Verfahren, Sauerstoff schneller im Blut angereichert und dem Blut schneller Kohlendioxid entzogen werden kann (Decarboxylierung). Hierdurch wird ein definierter Wechsel zwischen Überdruckbetrieb, d.h. einer schnellen Oxygenierung des Blutes zu Beginn der ECMO-Behandlung, und einem Unterdruckbetrieb, d.h. einem Konstanthalten des Sauerstoffpartialdrucks (bzw. der Menge des Sauerstoffs) im Blut auf einem physiologisch optimalen Wert im Verlauf der ECMO-Behandlung, möglich. Hierbei bezieht sich Über- bzw. Unterdruck auf physiologische Gasdruckwerte im Blut. Zudem ist die Decarboxylierung im Verlauf der ECMO-Behandlung gut steuerbar.
Aufgrund des (weitgehend) geschlossenen Gaskreislaufs ist das Verfahren unabhängig vom Umgebungsdruck, wobei der Druck und die Strömungsgeschwindigkeit des Prozessgases weitestgehend unabhängig voneinander eingestellt werden können, und der Sauerstoffverbrauch ist, im Gegensatz zu herkömmlichen Verfahren, sehr gering, da lediglich die Sauerstoffmenge nachgeführt wird, die vom Blut aufgenommen wurde. Infolgedessen entstehen wesentlich geringere Kosten und der Gewichts- und Platzbedarf der benötigten Sauerstoffversorgung (Gasflaschen) wird nachhaltig gesenkt, was insbesondere bei mobilen Anwendungen sehr vorteilhaft ist. Vorteilhaft ist auch, dass in der Umgebung keine Anreicherung von Sauerstoff erfolgt (Verhinderung der Brandgefahr).

Neben der Verwendung für die ECMO kann das Verfahren auch für andere Austauschsysteme (Gas/Gas; Gas/Flüssigkeit) angewendet werden, wie z.B. Gasaustauschverfahren von physiologischen und nicht physiologischen Gasen in Blut hinein (Narkosegas) und aus Blut heraus (Narkosegas oder toxische Gase). Zwar wurde das Verfahren als Beispiel vorstehend unter Bezugnahme auf Blut als Flüssigkeit, Sauerstoff als erstem Gas und Kohlendioxid als zweitem Gas erläutert; jedoch kann das beschriebene Verfahren allgemein zum Einbringen eines ersten Gases in eine Flüssigkeit unter Erhöhung der in der Flüssigkeit vorliegenden Menge des ersten Gases und zum Entfernen eines zweiten Gases aus der Flüssigkeit unter Verringerung der in der Flüssigkeit vorliegenden Menge des zweiten Gases verwendet werden, wobei die Menge des in die Flüssigkeit eingetragenen ersten Gases bzw. die Eintragrate mittels Einstellens des Drucks des Prozessgases (wobei das Prozessgas identisch mit dem ersten Gas sein kann) eingestellt wird, und wobei die Menge des aus der Flüssigkeit entfernten zweiten Gases bzw. die Austragsrate mittels Einstellens der Strömungsgeschwindigkeit des Prozessgases eingestellt wird.

Zur Regelung des Drucks des Prozessgases im Gaskreislauf wird, sobald eine bestimmte Verringerung des Gasdrucks im Gaskreislauf, z.B. mittels eines Drucksensors, festgestellt wird, so lange das erste Gas mittels eines Ventils, z.B. einer Druckregeleinheit, eines Gasregelventils oder eines Gashahns, dem Gaskreislauf zugeführt, bis ein zuvor festgelegter (definierter) Gasdruck im Prozessgas erreicht wird. Es kann auch eine Gasregeleinheit eingesetzt werden, bei der ein Gasregelventil und ein Drucksensor in einer Einheit zusammengefasst sind. Da bei dem Verfahren lediglich der Druck des Prozessgases und die Strömungsgeschwindigkeit des Prozessgases im Gaskreislauf variiert werden müssen, ist mit dem Verfahren eine sehr hohe Anwendungs- und Patientensicherheit erreichbar.

In einer einfachen Variante des Verfahrens wird die Einstellung der Mengen oder Partialdrücke des ersten und des zweiten Gases in der Flüssigkeit vom Anwender gesteuert, indem er die beiden Partialdrücke (oder Mengen) in der Flüssigkeit, z.B. durch eine Blutabnahme und Analyse des Sauerstoff- und Kohlendioxidpartialdrucks, ermittelt und bei Abweichungen von den vorgegebenen Werten entsprechend gegensteuert (durch Variation des Druckes des Prozessgases bei Abweichungen der Menge/des Partialdrucks des ersten Gases in der Flüssigkeit bzw. durch Variation der Strömungsgeschwindigkeit des Prozessgases im Gaskreislauf bei einer Abweichung der Menge/des Partialdrucks des zweiten Gases in der Flüssigkeit).
Alternativ werden die Mengen/die Partialdrücke des ersten Gases und des zweiten Gases in der Flüssigkeit mittels Sensoren gemessen. Mit den ermittelten Messwerten werden vollautomatisch mittels einer Regeleinheit der Druck des Prozessgases und die Strömungsgeschwindigkeit des Prozessgases derart eingestellt, dass die Menge/der Partialdruck des ersten Gases in der Flüssigkeit auf den ersten vorgegebenen Wert und die Menge/der Partialdruck des zweiten Gases in der Flüssigkeit auf den zweiten vorgegebenen Wert eingeregelt (mit Rückmeldung durch Sensor) oder eingestellt (ohne Rückmeldung durch Sensor) werden.
Demgemäß kann z.B. der vorliegende Ist-Wert der Menge oder des Partialdrucks des ersten Gases in der Flüssigkeit mittels eines entsprechenden Sensors erfasst werden, z.B. bevor und/oder nachdem die Flüssigkeit die Trennschicht kontaktiert. Gemäß dem Verfahren kann vorgesehen sein, den Druck des Prozessgases (welches das erste Gas enthält oder aus dem ersten Gas besteht) mittels Zuführens einer Menge des ersten Gases in den Gaskreislauf zu erhöhen, wenn der erfasste Ist-Wert der Menge oder des Partialdrucks des ersten Gases in der Flüssigkeit kleiner ist als ein zugehöriger Soll-Wert der Menge bzw. des Partialdrucks des ersten Gases in der Flüssigkeit. Alternativ oder zusätzlich kann vorgesehen sein, den Druck des Prozessgases (z.B. mittels Abpumpens von Prozessgas) aktiv zu verringern, wenn der erfasste Ist-Wert der Menge oder des Partialdrucks des ersten Gases in der Flüssigkeit größer ist als der zugehörige Soll-Wert. Das Abpumpen kann z.B. mittels einer an den Gaskreislauf angebundenen Unterdruck- bzw. Evakuierungspumpe erfolgen; wobei eine solche Evakuierungspumpe auch allgemein zum Bereitstellen eines Unterdrucks in dem Gaskreislauf vorgesehen sein kann (sodass z.B. während des Verfahrens ein Unterdruck, z.B. ein Druck von kleiner als 1 bar, in dem Gaskreislauf vorliegt).

Zudem kann z.B. der vorliegende Ist-Wert der Menge oder des Partialdrucks des zweiten Gases in der Flüssigkeit mittels eines entsprechenden Sensors erfasst werden, z.B. bevor und/oder nachdem die Flüssigkeit die Trennschicht kontaktiert. Gemäß dem Verfahren kann vorgesehen sein, die Strömungsgeschwindigkeit des Prozessgases in dem Gaskreislauf zu erhöhen, wenn der erfasste Ist-Wert der Menge oder des Partialdrucks des zweiten Gases in der Flüssigkeit größer ist als ein zugehöriger Soll-Wert der Menge bzw. des Partialdrucks des zweiten Gases in der Flüssigkeit. Alternativ oder zusätzlich kann vorgesehen sein, die Strömungsgeschwindigkeit des Prozessgases zu verringern, wenn der erfasste Ist-Wert der Menge oder des Partialdrucks des zweiten Gases in der Flüssigkeit kleiner ist als der zugehörige Soll-Wert.

Bei einer Erhöhung der Strömungs- bzw. Umwälzgeschwindigkeit im geschlossenen Gaskreislauf erhöht sich jedoch nicht nur die Abscheidung des zweiten Gases im Adsorbens (z.B. die Abscheidung des Kohlendioxids aus Blut), sondern ebenso der Übertritt des ersten Gases in die Flüssigkeit (z.B. der Übertritt von Sauerstoff in Blut). Um dem entgegenzuwirken, kann bei einer Erhöhung der Strömungsgeschwindigkeit des Prozessgases vorgesehen sein, den Druck des ersten Gases im Gaskreislauf (z.B. den Sauerstoffdruck) zu senken, so dass als Ergebnis der Einstellung bzw. Regelung der Gehalt des ersten Gases in der Flüssigkeit (z.B. der Blutsauerstoffgehalt) gleich bleibt.

Analog kann bei einer (zum Verringern der Abscheidung des zweiten Gases aus der Flüssigkeit erfolgenden) Verringerung der Strömungsgeschwindigkeit des Prozessgases vorgesehen sein, den Druck des ersten Gases im Gaskreislauf (z.B. den Sauerstoffdruck) derart zu erhöhen, dass als Ergebnis der Einstellung bzw. Regelung der Gehalt des ersten Gases in der Flüssigkeit (z.B. der Blutsauerstoffgehalt) gleich bleibt.

Bei einer zur Reduzierung des Eintrags des ersten Gases in die Flüssigkeit (z.B. zur Reduzierung des Blutsauerstoffgehalts) erfolgenden Senkung des Drucks des ersten Gases (z.B. des Sauerstoffdrucks) im Gaskreislauf sinkt auch der gesamte Gasdruck; wobei in einem solchen Fall zur Vermeidung einer zu starken Abreicherung des zweiten Gases in der Flüssigkeit (z.B. einer zu starken Kohlendioxid-Abreicherung des Bluts) vorgesehen sein kann, die Strömungs- bzw. Rezirkulationsgeschwindigkeit zu vermindern. Da dadurch jedoch auch der Übertritt des ersten Gases aus dem Prozessgas in die Flüssigkeit (z.B. der Übertritt von Sauerstoff in das Blut) verringert wird, muss der Druck in der Regelungspraxis weniger stark gesenkt werden als bei einer allein über den Druck erfolgenden Regelung. Entsprechendes gilt für eine Drucksteigerung; d.h., bei einer Erhöhung des Drucks des ersten Gases im Gaskreislauf kann vorgesehen sein, zugleich die Strömungsgeschwindigkeit des Prozessgases zu erhöhen. Die Kombination aus Druck- und Flowregelung erhöht auf diese Weise die Regeldynamik.

Die Vorrichtung zur Durchführung des Verfahrens weist eine Trennschicht, deren eine Seite beim Betrieb der Vorrichtung von einem Prozessgas und deren andere Seite von einer Flüssigkeit hinterströmt ist, und einen (geschlossenen) Kreislauf für das Prozessgas mit einer Pumpe bzw. Umwälzpumpe, die zum Umwälzen des Prozessgases im Kreislauf dient, mindestens eine Absorbereinheit, die dem Prozessgas das zweite Gas entziehen und aus dem Kreislauf entfernen kann, und mindestens eine über ein Ventil gesteuerte Nachführung des ersten Gases auf. Die Gasnachführung bzw. Gaszuführeinrichtung ist somit mittels eines einstellbaren Ventils an den Gaskreislauf angekoppelt. Die Vorrichtung kann ferner eine an den Gaskreislauf angebundene Evakuierungspumpe (die z.B. Bestandteil einer Purgeeinrichtung der Vorrichtung ist) zum Herauspumpen von Prozessgas aus dem Gaskreislauf aufweisen.

Die Vorrichtung weist flüssigkeitsseitig einen ersten Sensor zum Erfassen der Menge oder des Partialdrucks des ersten Gases in der Flüssigkeit sowie einen zweiten Sensor zum Erfassen der Menge oder des Partialdrucks des zweiten Gases in der Flüssigkeit auf. Die Vorrichtung kann zudem prozessgasseitig einen Drucksensor zum Erfassen des Drucks des Prozessgases in dem Gaskreislauf und einen Strömungsgeschwindigkeitssensor zum Erfassen der Strömungsgeschwindigkeit des Prozessgases in dem Gaskreislauf aufweisen.
Des Weiteren kann die Vorrichtung eine Steuereinheit aufweisen, die mit dem Ventil, der Evakuierungspumpe und der Umwälzpumpe verbunden ist und derart konfiguriert ist, dass von ihr mittels Ansteuerns des Ventils und der Evakuierungspumpe der Druck des Prozessgases in dem Gaskreislauf und mittels Ansteuerns der Umwälzpumpe die Strömungsgeschwindigkeit des Prozessgases in dem Gaskreislauf einstellbar ist. Erfindungsgemäß weist die Vorrichtung eine Regeleinheit auf, die mit dem flüssigkeitsseitigen ersten Sensor, dem flüssigkeitsseitigen zweiten Sensor, dem prozessgasseitigen Drucksensor, dem prozessgasseitigen Strömungsgeschwindigkeitssensor, dem Ventil, der Evakuierungspumpe und der Umwälzpumpe verbunden ist und derart konfiguriert ist, dass von ihr basierend auf dem Messsignal des ersten Sensors mittels Ansteuerns des Ventils und der Evakuierungspumpe der Druck des Prozessgases in dem Gaskreislauf geregelt einstellbar ist, und dass von ihr basierend auf dem Messsignal des zweiten Sensors mittels Ansteuerns der Umwälzpumpe die Strömungsgeschwindigkeit des Prozessgases in dem Gaskreislauf geregelt einstellbar ist.
Die Steuerung bzw. Regelung des Drucks und der Strömungsgeschwindigkeit des Prozessgases erfolgen wie mit Bezug auf das Verfahren erläutert. So ist vorgesehen, mittels Aufdrehens bzw. Öffnens des Ventils den Druck des Prozessgases zu erhöhen, wenn der mittels des ersten Sensors erfasste Ist-Wert der Menge bzw. des Partialdrucks des ersten Gases in der Flüssigkeit unterhalb eines zugehörigen vorgegebenen Soll-Werts liegt. Zusätzlich ist vorgesehen, mittels Zudrehens des Ventils und/oder Abpumpens von Prozessgas den Druck des Prozessgases zu verringern, wenn der mittels des ersten Sensors erfasste Ist-Wert oberhalb des zugehörigen Soll-Werts liegt.

Zudem ist vorgesehen, mittels Beschleunigens des Pumpvorgangs die Strömungsgeschwindigkeit des Prozessgases zu erhöhen, wenn der mittels des zweiten Sensors erfasste Ist-Wert der Menge bzw. des Partialdrucks des zweiten Gases in der Flüssigkeit oberhalb eines zugehörigen vorgegebenen Soll-Werts liegt. Zusätzlich ist vorgesehen, mittels Verlangsamens des Pumpvorgangs die Strömungsgeschwindigkeit des Prozessgases zu reduzieren, wenn der mittels des zweiten Sensors erfasste Ist-Wert der Menge bzw. des Partialdrucks des zweiten Gases in der Flüssigkeit unterhalb des zugehörigen Soll-Werts liegt.
Das jeweilige Einstellen des Drucks basierend auf dem Messsignal des ersten Sensors und der Strömungsgeschwindigkeit basierend auf dem Messsignal des zweiten Sensors können mittels der Ankopplung des prozessgasseitigen Drucksensors und Strömungsgeschwindigkeitssensors an die Regeleinheit geregelt erfolgen.
Falls die Flüssigkeit Wasser enthält, kann ein Übertritt von Wasser (z.B. in Form von Wasserdampf) aus der Flüssigkeit über die permeable Trennschicht in den Gaskreislauf erfolgen. Um eine Anreicherung von Wasserdampf und die damit verbundene Änderung der Mengen/der Partialdrücke des ersten und zweiten Gases (z.B. Sauerstoff und Kohlendioxid) zu vermeiden, kann im Kreislauf eine Einrichtung zur Entfernung von Wasserdampf aus dem Prozessgas, z.B. ein Gastrockner, angeordnet sein.
Durch große Mengen Wasserdampf im Gaskreislauf kann der Partialdruck der anderen Gase im Gaskreislauf unerwünscht absinken. Insbesondere kann dadurch die Nachführung des Gehalts des ersten Gases in der Flüssigkeit (z.B. des Blutsauerstoffwertes) über die Druckregelung beeinträchtigt sein, da der Gasdruck (als Summe der Partialdrücke) in diesem Falle steigt, auch wenn immer weniger erstes Gas (z.B. Sauerstoff) nachgeführt und immer mehr zweites Gas (z.B. Kohlendioxid) abgeschieden wird. Das Entfernen von Störgasen durch gelegentliches Spülen (Purgen) weist den Nachteil auf, dass für eine ausreichende Entfernung des Wasserdampfs nahezu das gesamte Gaskreislaufvolumen gepurged und anschließend aus dem normalen Regelprozessablauf wieder ergänzt werden muss. Zudem wird so die gewünschte Gasersparnis zum Teil zunichte gemacht und die Regelung zwischen zwei Spülvorgängen ist weiterhin störbehaftet. Des Weiteren ist oft ein gewisser Wasserdampfdruck erforderlich, um die Trennschicht bzw. Membran und andere Funktionselemente der Vorrichtung zu befeuchten.

Bei Blut als Flüssigkeit und Sauerstoff als erstem Gas, wobei die Vorrichtung also als Oxygenator ausgebildet ist, ist bei Betrieb des Oxygenators mit geringem Gasdruck, namentlich bei Drücken unterhalb des Blutdrucks, ganz besonders aber bei Drücken unterhalb des Atmosphärendrucks, mit einem im Vergleich zum Überdruckbetrieb stärkeren Übertritt von Wasserdampf oder sogar flüssigem Wasser von der Blut- auf die Gasseite des Oxygenators zu rechnen.

Der Wasserdampf wird in einem solchen Fall mittels einer Trocknungseinrichtung - z.B. mittels einer Kühlfalle, eines Adsorbens oder eines Gastrockners (bspw. Nafion-Membran-Entfeuchter) - im Gaskreislauf auf einen wünschenswerten bzw. vorgegebenen Partialdruck eingestellt. Die Trocknungseinrichtungen können durch eine Einstellmöglichkeit in ihrer Wirkung variiert werden (beim Kondensor/Kühlfalle vorzugsweise durch Einstellen der Temperatur, beim Membran-Gastrockner durch Variation des Trocknungsgasstromes, bei allen Einrichtungen gleichermaßen durch partielle oder periodische Vorbeileitung (Bypass) des zirkulierenden Gases. Nach Literaturangaben muss man damit rechnen, dass als Beispiel bei 1,9 m² Oxygenatorfläche alle 7 Stunden ein Mol Wasserdampf (-24 Milliliter) über die Membran auf die Gasseite gelangt. Es stellt sich freilich immer von alleine die Sättigungsfeuchte für die jeweils kühlste Stelle der Anlage ein. Dies sollte jedoch keinesfalls einfach irgendwo erfolgen und sollte regelungstechnisch berücksichtigt werden. Es kann insbesondere vorgesehen sein, zumindest die Temperatur an der kältesten von dem Prozessgas umströmten Position der Vorrichtung zu erfassen (z.B. mittels eines an dem entsprechenden Bauelement der Vorrichtung positionierten Temperatursensors).

Der im Blut eventuell gelöste Stickstoff ist zwar physiologisch von geringerer Bedeutung, aber bei Anschluss an einen mit reinem Sauerstoff betriebenen Oxygenator mit geschlossenem Gaskreislauf würde sich dieser zunächst dort anreichern, bis das Blut annähernd stickstofffrei ist. Da Stickstoff auch im Gewebe löslich ist, muss man nicht nur das Blutvolumen, sondern auch das Zellvolumen für eine Berechnung der zu erwartenden Stickstoffmenge ansetzen. Deshalb muss zu Beginn der Oxygenierung öfter gespült werden.

Um Fremdgase vor jeder Inbetriebnahme und bei Bedarf intermittierend aus dem Gaskreislauf zu entfernen, kann in die Vorrichtung eine Purgeeinrichtung (Spüleinrichtung) integriert sein, die ein Durchspülen des Gaskreislaufs, z.B. mit Inert- oder Prozessgas, ermöglicht.

Wird zur Regelung des Gehalts des ersten Gases in der Flüssigkeit (z.B. zur Regelung des Blutsauerstoffgehalts) erfindungsgemäß der Partialdruck des ersten Gases (z.B. der Sauerstoffpartialdruck) im Gaskreislauf geändert, so ergibt sich mitunter die Notwendigkeit, sehr rasche Druckänderungen vornehmen zu müssen. Während eine rasche Steigerung durch einfaches Zuführen großer Mengen des ersten Gases (z.B. großer Sauerstoffmengen) leicht erzielt werden kann, so ist eine rasche Drucksenkung allein auf Basis des Gasübertritts in die Flüssigkeit nicht zu erwarten. Die Purgepumpe oder eine andere an den Gaskreislauf angebundene Evakuierungspumpe kann in diesem Fall für eine (rasche) Drucksenkung im Gaskreislauf verwendet werden; wobei der Druck im Gaskreislauf z.B. mittels einer solchen Pumpe allgemein auf einem Unterdruck gehalten werden kann. Bevorzugt ist die Vorrichtung mittels einer entsprechenden Druckregeleinrichtung derart ausgebildet, dass der Druck im Gaskreislauf mit einem Gradienten zwischen 50 mbar und 1000 mbar pro Sekunde in einem bevorzugten Druckbereich zwischen 50 mbar und 10 bar geändert werden kann.

Die Vorrichtung ist bevorzugt derart ausgebildet, dass die Regelpräzision und Regelgeschwindigkeit der Druckregeleinrichtung ein Vielfaches der Regelpräzision und Regelgeschwindigkeit der anderen Stellglieder wie z.B. Umwälzpumpe oder Kondensator beträgt. Durch eine Regelgeschwindigkeit zwischen 50 mbar und 10 bar pro Sekunde, besonders bevorzugt 10 mbar bis 1 bar pro Sekunde, und eine gleichzeitig hohe Regelpräzision von bevorzugt 0,01 bis 50 mbar, besonders bevorzugt 0,1 bis 10 mbar, kann ein durch die geringe Regelpräzision der anderen Stellglieder verursachtes Schwanken des Gehalts des ersten Gases in der Flüssigkeit (z.B. des Blutsauerstoffgehalts) rasch durch eine schnelle und präzise, den Schwankungen entgegengesetzte, Druckänderung ausgeglichen werden.

Durch gegebenenfalls notwendiges Spülen (Purgen) entstünden unerwünschte Drucksprünge, die eine plötzliche Änderung des Gehalts des ersten Gases in der Flüssigkeit (z.B. des Blutsauerstoffgehalts) und große mechanische Druckwechselbelastungen in der Vorrichtung (die z.B. als Oxygenator ausgebildet ist) zur Folge hätten. Durch die erläuterte hohe Regelgeschwindigkeit kann ein solcher pumpenbedingter Druckeinbruch nahezu vollständig ausgeregelt werden. Gleiches gilt für beim Verstellen oder Schalten von im Gaskreislauf befindlichen Ventilen entstehende Druckstöße.

Die Druckregeleinrichtung kann mittels Stell- und Regelventilen unterschiedlichster Bauart realisiert sein. Sinnvoll besonders für portable Anwendungen sind jedoch allein Verwirklichungsformen, in welchen die Bauteile der Gasdruckregelung weniger als 20% zum Anlagengewicht und Volumen beitragen. Bevorzugt soll daher das erste Gas (z.B. der Sauerstoff) dem Gaskreislauf in diskreten Portionen von 1 µL bis 10 ml unter Verwendung eines Schaltventils hinzugefügt werden. Besonders bevorzugt kann dieses Schaltventil die Druckdifferenz zwischen dem zum Vorhalten des ersten Gases vorgesehenen Vorratsgefäß (z.B. dem Sauerstoff-Vorratsgefäß) und dem Druck im Gaskreislauf bzw. Oxygenator einstufig überwinden und sperrt im geschlossenen Zustand den Gasstrom im technischen Sinne dicht ab.

Mittels der beschriebenen großen Dynamik und Stellgeschwindigkeit kann der Gasdruckregler, mit dem das erste Gas zugeführt wird, zur Erzeugung einer Pulsation bzw. eines Pulsierens des Gasdrucks verwendet werden (d.h. zum Erzeugen periodischer Druckschwankungen in dem Prozessgas). Bevorzugt ist die Pulsation so schnell, dass sie keinen spürbaren Einfluss auf den Gehalt des ersten und des zweiten Gases in der Flüssigkeit (z.B. auf den Blutsauerstoff- und Kohlendioxydgehalt) hat, aber durch Druckwechsel die Austauschprozesse an der Trennschicht bzw. Membran begünstigt und z.B. im Falle von Blut als Flüssigkeit und Sauerstoff als erstem Gas durch Übertrag der Druckstöße über eine flexible Membran ins Blut die physiologischen Prozesse im oxygenierten Organismus positiv beeinflusst.

Bei einer Verwendung der Vorrichtung für die ECMO kann als Absorbereinheit ein Kohlendioxid-Absorber, z.B. basierend auf Kalk, eingesetzt sein.

Die Erfindung wird nachfolgend anhand zweier Ausführungsbeispiele näher erläutert; hierzu zeigen:
- Fig. 1:: Schaltbild einer Vorrichtung für ECMO-Anwendungen mit Steuerung durch den Anwender;
- Fig. 2:: Schaltbild einer Vorrichtung für ECMO-Anwendungen mit vollautomatischer Regelung.

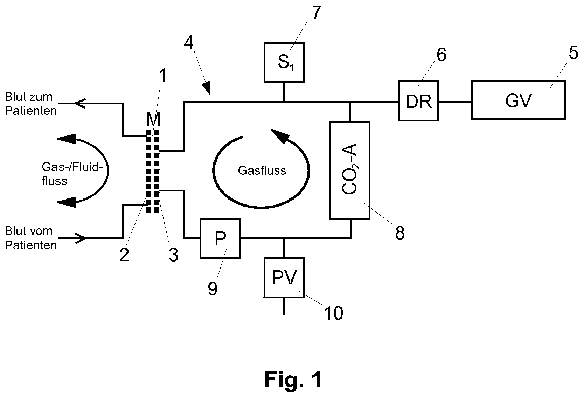

Die in Fig. 1 dargestellte ECMO-Vorrichtung arbeitet halbautomatisch, d.h., die Regelung der Menge/des Partialdrucks von Sauerstoff und des Partialdrucks von Kohlendioxid im Blut des behandelten Patienten wird vom Anwender vorgenommen. Die ECMO-Vorrichtung besteht aus einer Membran 1, an deren einen Seite 2 das Blut des behandelten Patienten und an deren anderen Seite 3 ein in einem Gaskreislauf 4 geführtes Prozessgas vorbeigeleitet wird. Das Prozessgas gibt beim Betrieb der Vorrichtung einerseits Sauerstoff an das Blut ab und nimmt anderseits Kohlendioxid aus dem Blut auf, wodurch die ständige Verringerung der Sauerstoffmenge/des Sauerstoffpartialdrucks und die Erhöhung des Kohlendioxidpartialdrucks im Körper des Patienten (aufgrund von Stoffwechselvorgängen) ausgeglichen wird.

Der Gaskreislauf umfasst, neben der Membran 1 (bzw. deren Prozessgasseite 3), eine Gasversorgung 5, die über eine Druckregeleinheit 6 an den Gaskreislauf 4 angebunden ist, einen Drucksensor 7, einen Kohlendioxidabsorber 8, mit dem dem Gaskreislauf 4 das über die Membran 1 ins Prozessgas gelangte Kohlendioxid entzogen wird, eine Gaspumpe 9, die zur Umwälzung des Prozessgases im Gaskreislauf 4 dient, und eine Purgeeinrichtung mit einem Purgeventil 10, mit dem der Gaskreislauf 4, zur Entfernung von Fremdgasen, vor jeder Inbetriebnahme gespült werden kann. Durch das Purgeventil 10 hindurch kann zudem zum Verringern des in dem Gaskreislauf vorliegenden Drucks Prozessgas aus dem Gaskreislauf 4 abgepumpt werden; z.B. indem das Purgeventil 10 mit einer Evakuierungspumpe der Purgeeinrichtung oder einer sonstigen Vakuum-Leitung verbunden ist. Dadurch kann die ECMO-Vorrichtung insbesondere zum Betrieb unterhalb des Atmosphärendrucks ausgebildet sein, wobei der in dem Gaskreislauf 4 vorliegende Druck kleiner als der umgebende Atmosphärendruck ist. Die Druckregeleinheit 6 überwacht den Druck im Gaskreislauf 4 mittels des Drucksensors 7 und speist, sobald der Druck, aufgrund der Sauerstoffabgabe aus dem Prozessgas in das Blut des behandelten Patienten, unter einen festgelegten Wert gefallen ist, Sauerstoff in den Gaskreislauf 4 nach (bis der Wert wieder überschritten ist). Der Kohlendioxidabsorber 8 kann optional zusätzlich als Trocknungseinrichtung zum Entfernen von Wasserdampf aus dem Gasstrom und Einstellen des in dem Gaskreislauf vorliegenden Wasserdampfgehalts auf einen vorgegebenen Partialdruck ausgebildet sein.

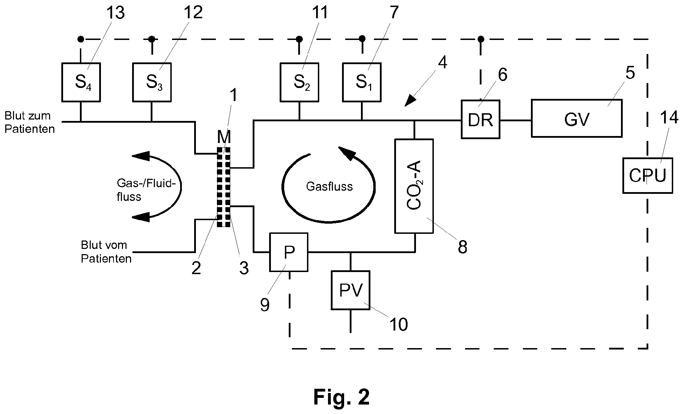

Die ECMO-Vorrichtung zur vollautomatischen Regelung der Menge/des Partialdrucks von Sauerstoff und des Partialdrucks von Kohlendioxid im Blut des behandelten Patienten (Fig. 2) ist analog zur ECMO-Vorrichtung für den halbautomatischen Betrieb aufgebaut, weist jedoch zusätzlich einen Partialdrucksensor oder optischen Sensor für Sauerstoff 12, einen Partialdrucksensor für Kohlendioxid oder pH-Sensor 13 und eine Steuereinheit 14 auf. Die Steuereinheit 14 regelt anhand der Messwerte des Partialdrucksensors/des optischen Sensors für Sauerstoff 12 (über den Druck des Prozessgases) die Menge des Sauerstoffs/den Sauerstoffpartialdruck im Blut und anhand der Messwerte des Partialdrucksensors/pH-Sensors 13 (über die Strömungsgeschwindigkeit des Prozessgases im Gaskreislauf 4), den Kohlendioxidpartialdruck im Blut des Patienten ein.

### Liste der verwendeten Bezugszeichen

- 1: Trennschicht/Membran
- 2: Seite der Membran, die mit Blut in Kontakt steht
- 3: Seite der Membran, die mit Prozessgas in Kontakt steht
- 4: Gaskreislauf
- 5: Gasversorgung
- 6: Ventil, Druckregeleinheit
- 7: Drucksensor
- 8: Absorbereinheit für das zweite Gas/Kohlendioxidabsorber
- 9: Pumpe/Gaspumpe
- 10: Purgeventil
- 11: Drucksensor
- 12: Partialdrucksensor/optischer Sensor für Sauerstoff/für das erste Gas
- 13: Partialdrucksensor/pH-Sensor für Kohlendioxid/für das zweite Gas
- 14: Steuereinheit

## Patentansprüche

1. Vorrichtung zur Einstellung der Menge oder des Partialdruckes eines ersten Gases auf einen ersten vorgegebenen Wert und eines zweiten Gases auf einen zweiten vorgegebenen Wert in Blut, aufweisend eine Trennschicht (1), deren eine Seite (3) beim Betrieb der Vorrichtung von einem Prozessgas und deren andere (2) Seite von dem Blut hinterströmt ist, einen geschlossenen Gaskreislauf (4) für das Prozessgas mit einer Pumpe (9), die zum Umwälzen des Prozessgases im Gaskreislauf (4) dient, mindestens einer Absorbereinheit (8), die dem Prozessgas das zweite Gas entziehen und aus dem Kreislauf entfernen kann, und mindestens eine mittels eines Ventils (6) an den Gaskreislauf angekoppelte Gasversorgung zum Zuführen des ersten Gases, einen ersten Sensor zum Erfassen der Menge oder des Partialdrucks des ersten Gases in dem Blut, einen zweiten Sensor zum Erfassen der Menge oder des Partialdrucks des zweiten Gases in dem Blut, und eine Regeleinheit zum Einstellen des Drucks des Prozessgases anhand der Messwerte des ersten Sensors und Einstellen der Strömungsgeschwindigkeit des Prozessgases anhand der Messwerte des zweiten Sensors, wobei die Regeleinheit eingerichtet ist, mittels Aufdrehens des Ventils den Druck des Prozessgases zu erhöhen, wenn der mit dem ersten Sensor erfasste Ist-Wert der Menge oder des Partialdrucks des ersten Gases im Blut unterhalb eines zugehörigen vorgegebenen Soll-Werts liegt, mittels Zudrehens des Ventils und/oder Abpumpens von Prozessgas den Druck des Prozessgases zu verringern, wenn der mit dem ersten Sensor erfasste Ist-Wert oberhalb des zugehörigen Soll-Werts liegt, mittels Beschleunigens des Pumpvorgangs die Strömungsgeschwindigkeit des Prozessgases zu erhöhen, wenn der mittels des zweiten Sensors erfasste Ist-Wert der Menge oder des Partialdrucks des zweiten Gases in dem Blut oberhalb eines zugehörigen vorgegebenen Soll-Werts liegt und mittels Verlangsamens des Pumpvorgangs die Strömungsgeschwindigkeit des Prozessgases zu reduzieren, wenn der mittels des zweiten Sensors erfasste Ist-Wert der Menge oder des Partialdrucks des zweiten Gases in dem Blut unterhalb des zugehörigen Soll-Werts liegt.

2. Vorrichtung nach Anspruch 1, **dadurch gekennzeichnet, dass** im Kreislauf für das Prozessgas als Pumpe (9) eine Gaspumpe eingesetzt ist.

3. Vorrichtung nach einem der Ansprüche 1 oder 2, **dadurch gekennzeichnet, dass** im Kreislauf eine Einrichtung zur Entfernung von Wasserdampf aus dem Prozessgas angeordnet ist.

4. Vorrichtung nach einem der Ansprüche 1 bis 3, **dadurch gekennzeichnet, dass** im Kreislauf für das Prozessgas eine Purgeeinrichtung integriert ist, die es ermöglicht, intermittierend Fremdgase aus dem Prozessgas über ein Purgeventil (10) aus dem Kreislauf zu entfernen und zu Betriebsbeginn die Vorrichtung mit Prozessgas zu fluten.

5. Vorrichtung nach einem der Ansprüche 1 bis 4, **dadurch gekennzeichnet, dass** der erste Sensor ein optischer Sensor oder ein Partialdrucksensor zur Detektion von Sauerstoff ist.

6. Vorrichtung nach einem der Ansprüche 1 bis 5, **dadurch gekennzeichnet, dass** der zweite Sensor ein pH-Sensor oder ein Partialdrucksensor zur Detektion von Kohlendioxid und die Absorbereinheit (8) ein Kohlendioxid-Absorber ist.

7. Vorrichtung nach einem der Ansprüche 1 bis 6, **dadurch gekennzeichnet, dass** die Regeleinheit eine Regelpräzision von 0,1 bis 10 mbar und eine Regelgeschwindigkeit zwischen 10 mbar bis 1 bar pro Sekunde aufweist.

8. Vorrichtung nach einem der Ansprüche 1 bis 7, **dadurch gekennzeichnet, dass** eine Evakuierungspumpe mit dem geschlossenen Gaskreislauf (4) für das Prozessgas verbunden ist, wodurch dieser unterhalb des Atmosphärendrucks betreibbar ist.

## Claims

1. A device for adjusting the amount or the partial pressure of a first gas to a first predefined value and of a second gas to a second predefined value in blood, comprising a separating layer (1), on one side (3) of which a process gas flows during operation of the device and on the other side (2) of which blood flows during operation of the device, and comprising a closed gas circuit (4) for the process gas with a pump (9), which is used to recirculate the process gas in the gas circuit (4), at least one absorber unit (8), which can extract the second gas from the process gas and remove it from the circuit, and at least one gas supply coupled by means of a valve (6) to the gas circuit for feeding the first gas, a first sensor for detecting the amount or the partial pressure of the first gas in the blood, a second sensor for detecting the amount or the partial pressure of the second gas in the blood, and a control unit for adjusting the pressure of the process gas on the basis of the measured values of the first sensor and for adjusting the flow rate of the process gas on the basis of the measured values of the second sensor, wherein the control unit is designed to increase the pressure of the process gas by opening the valve when the actual value of the amount or partial pressure of the first gas in the blood detected by the first sensor lies below an associated predefined target value, to reduce the pressure of the process gas by closing the valve and/or pumping off process gas when the actual value detected by the first sensor lies above the associated target value, to increase the flow rate of the process gas by accelerating the pumping operation when the actual value of the amount or partial pressure of the second gas in the blood detected by means of the second sensor lies above an associated predefined target value, and to reduce the flow rate of the process gas by slowing the pumping operation when the actual value of the amount or partial pressure of the second gas in the blood detected by means of the second sensor lies below the associated target value.

2. The device according to claim 1, **characterised in that** a gas pump is used as pump (9) in the circuit for the process gas.

3. The device according to either one or claims 1 or 2, **characterised in that** a means for removing steam from the process gas is arranged in the circuit.

4. The device according to any one of claims 1 to 3, **characterised in that** a purge means is integrated in the circuit for the process gas and makes it possible to remove foreign gases from the process gas intermittently via a purge valve (10) and to remove said gases from the circuit and to flood the device with process gas when operation is started.

5. The device according to any one of claims 1 to 4, **characterised in that** the first sensor is an optical sensor or a partial pressure sensor for detecting oxygen.

6. The device according to any one of claims 1 to 5, **characterised in that** the second sensor is a pH sensor or a partial pressure sensor for detecting carbon dioxide and the absorber unit (8) is a carbon dioxide absorber.

7. The device according to any one of claims 1 to 6, **characterised in that** the control unit has a control precision of from 0.1 to 10 mbar and a control speed of between 10 mbar and 1 bar per second.

8. The device according to any one of claims 1 to 7, **characterised in that** an evacuation pump is connected to the closed gas circuit (4) for the process gas, whereby this circuit can be operated beneath atmospheric pressure.

## Revendications

1. Dispositif de réglage de la quantité ou de la pression partielle d'un premier gaz à une première valeur prédéterminée et d'un deuxième gaz à une deuxième valeur prédéterminée dans du sang, présentant une couche de séparation (1), dont un côté (3), lors du fonctionnement du dispositif, est balayé par l'arrière par un gaz de processus, et dont l'autre côté (2) est balayé par l'arrière par le sang, un circuit de gaz (4) fermé pour le gaz de procédé avec une pompe (9), qui sert à inverser le gaz de procédé dans le circuit de gaz (4), au moins une unité d'absorption (8), qui peut prélever le deuxième gaz dans le gaz de procédé et l'éloigner du circuit, et au moins une alimentation de gaz couplée au circuit de gaz au moyen d'une vanne (6) pour l'approvisionnement du premier gaz, un premier capteur pour la détection de la quantité ou de la pression partielle du premier gaz dans le sang, un deuxième capteur pour la détection de la quantité ou de la pression partielle du deuxième gaz dans le sang, et une unité de commande pour le réglage de la pression du gaz de procédé à l'aide des valeurs de mesure du premier capteur et pour le réglage de la vitesse d'écoulement du gaz de procédé à l'aide des valeurs de mesure du deuxième capteur, où l'unité de commande est conçue pour, au moyen de l'ouverture par rotation de la vanne, augmenter la pression du gaz de procédé, si la valeur réelle de la quantité ou de la pression partielle du premier gaz dans le sang, détectée avec le premier capteur, se situe en dessous d'une valeur souhaitée prédéterminée correspondante, diminuer la pression du gaz de procédé au moyen de la fermeture par rotation de la vanne et/ou par pompage du gaz de procédé, si la valeur réelle détectée avec le premier capteur se situe au-dessus de la valeur souhaitée correspondante, augmenter la vitesse d'écoulement du gaz de procédé au moyen d'une accélération du processus de pompage, si la valeur réelle de la quantité ou de la pression partielle du deuxième gaz dans le sang, détectée au moyen du deuxième capteur, se situe au-dessus d'une valeur souhaitée prédéterminée correspondante et, au moyen d'un ralentissement du processus de pompage, réduire la vitesse d'écoulement du gaz de procédé, si la valeur réelle de la quantité ou de la pression partielle du deuxième gaz dans le sang, détectée au moyen du deuxième capteur, se situe en dessous de la valeur souhaitée prédéterminée correspondante.

2. Dispositif selon la revendication 1, **caractérisé en ce que**, dans le circuit pour le gaz de procédé, une pompe à gaz est employée en tant que pompe (9).

3. Dispositif selon l'une des revendications 1 ou 2, **caractérisé en ce que**, dans le circuit, une installation destinée au retrait de la vapeur d'eau à partir du gaz de procédé est disposée.

4. Dispositif selon l'une des revendications 1 à 3, **caractérisé en ce que**, dans le circuit, une installation de purge est intégrée pour le gaz de procédé, qui permet de retirer du circuit de manière intermittente des gaz étrangers à partir du gaz de procédé par l'intermédiaire d'une vanne de purge (10) et pour inonder le dispositif avec du gaz de procédé en début de fonctionnement.

5. Dispositif selon l'une des revendications 1 à 4, **caractérisé en ce que** le premier capteur est un capteur optique ou un capteur de pression partielle pour la détection d'oxygène.

6. Dispositif selon l'une des revendications 1 à 5, **caractérisé en ce que** le deuxième capteur est un capteur de pH ou un capteur de pression partielle destiné à la détection de dioxyde de carbone et que l'unité d'absorption (8) est un absorbeur de dioxyde de carbone.

7. Dispositif selon l'une des revendications 1 à 6, **caractérisé en ce que** l'unité de commande présente une précision de réglage de 0,1 à 10 mbar et une vitesse de réglage entre 10 mbar et 1 bar par seconde.

8. Dispositif selon l'une des revendications 1 à 7, **caractérisé en ce qu'**une pompe d'évacuation est reliée avec le circuit de gaz fermé (4) pour le gaz de procédé, ce par quoi celui-ci peut être déplacé en dessous de la pression atmosphérique.
